# FASCICULE DE BREVET EUROPEEN

(11) **EP 1 059 071 B1**
(45) Date de publication et mention de la délivrance du brevet: **01.09.2004**
(21) Numéro de dépôt: 00420116.6
(22) Date de dépôt: 07.06.2000
(51) Int. Cl.: A61F 2/40

(54) **Prothèse humérale**
Humerusprothese
Humeral prosthesis

(30) Priorité: 09.06.1999 FR 9907510
(43) Date de publication de la demande: 13.12.2000
(73) Titulaire: TORNIER SA, 38330 Saint-Ismier (FR)
(72) Inventeur: Tornier, Alain, 38330 Saint Ismier (FR); Boileau, Pascal, 06200 Nice (FR); Walch, Gilles, 69003 Lyon (FR)
(74) Mandataire: Myon, Gérard Jean-Pierre

(56) Documents cités:
- EP-A- 0 664 108
- EP-A- 0 821 924
- WO-A-94/15551
- WO-A-95/22302
- WO-A-98/15241
- FR-A- 2 755 847
- FR-A- 2 763 501
- GB-A- 1 292 561
- US-A- 5 282 865

## Description

La présente invention a trait à une prothèse humérale. Une prothèse selon le préambule est connue du document WO-A-95/22302.

Lors de la fracture de l'extrémité haute d'un humérus, ce dernier se brise généralement en plusieurs morceaux, à savoir la diaphyse, les tubérosités humérales et enfin la tête de l'humérus, qui coopère avec la glène de l'épaule.

Lors de la réduction d'une telle fracture, seule la tête humérale doit être remplacée par une calotte sensiblement hémisphérique, alors que les autres morceaux fracturés peuvent être reconstruits autour d'une prothèse.

De manière connue, une telle prothèse comprend généralement une queue, ou partie diaphysaire, introduite dans le canal huméral de la diaphyse de l'os. Cette queue est prolongée par une partie métaphysaire recourbée vers le haut et vers l'intérieur, en faisant référence à une prothèse portée par un patient debout, autour de laquelle sont rassemblées les tubérosités constituant la métaphyse de l'os, en vue de leur reconstruction. La partie métaphysaire de la prothèse se raccorde à une collerette de réception de la calotte remplaçant la tête humérale.

Ce type de prothèse présente cependant un inconvénient, en ce sens qu'elle ne permet pas un positionnement optimal des grande et petite tubérosités, en vue de leur suture.

Afin de remédier à cet inconvénient, on connaît, de WO-A-98/15241, une prothèse humérale pourvue de moyens de positionnement de ces tubérosités humérales. A cet effet, la zone de raccord entre la partie métaphysaire de la prothèse et la collerette est pourvue, dans sa partie externe, d'au moins une nervure s'étendant, par rapport au plan de symétrie de cette prothèse, selon un angle compris entre 20 et 40°. Une telle nervure, qui peut être dédoublée, s'étend en direction de la gouttière bicipitale, au voisinage de laquelle les grande et petite tubérosités doivent être réunies. Cependant, une telle solution n'est pas complètement satisfaisante sur le plan anatomique, car la mise en place des tubérosités humérales ne peut pas être effectuée de façon certaine dans leur configuration naturelle.

Afin de pallier l'ensemble des inconvénients de l'art antérieur évoqués ci-dessus, l'invention se propose de réaliser une prothèse humérale permettant un positionnement précis et anatomique des grande et petite tubérosités, en vue de leur solidarisation.

Dans cet esprit, l'invention concerne une prothèse humérale comprenant une queue destinée à être ancrée dans le canal médullaire d'un humérus, une partie métaphysaire prolongeant cette queue vers le haut et dont la portion interne est inclinée vers l'intérieur, cette partie métaphysaire se raccordant à une collerette de support d'une calotte apte à coopérer avec la glène de l'épaule, caractérisée en ce que la portion externe de la partie métaphysaire est également inclinée vers l'intérieur, et cette portion externe comprend au moins un aileron antéro-postérieur d'appui des tubérosités humérales, le ou chaque aileron s'étendant dans un plan orienté selon un angle compris entre 45 et 135° par rapport au plan frontal de la prothèse.

Grâce à l'invention, et en particulier à l'orientation et aux dimensions du ou des ailerons antéro-postérieurs, une reconstruction des tubérosités humérales peut avoir lieu autour de la portion externe de la partie métaphysaire de la prothèse dans une configuration satisfaisante du point de vue anatomique. On peut, en particulier, prévoir que l'angle d'orientation du plan du ou des ailerons antéro-postérieurs par rapport au plan frontal de la prothèse est voisin de 90°. Cette configuration s'avère particulièrement avantageuse pour la reconstruction de la métaphyse humérale.

Selon des aspects avantageux de l'invention, la prothèse incorpore une ou plusieurs des caractéristiques suivantes :
- Le ou chaque aileron antéro-portérieur s'étend, à partir du plan frontal, avec une largeur maximale telle que sa projection sur un plan perpendiculaire au plan frontal correspond sensiblement à la distance séparant le bord postérieur de la gouttière bicipitale de la métaphyse humérale par rapport à un axe central de la tête humérale. La largeur du ou des ailerons antéro-postérieurs est ainsi adaptée à l'anatomie de la métaphyse humérale.
- La partie métaphysaire comprend en outre, dans sa portion externe, un aileron médian externe s'étendant globalement selon ledit plan frontal, le ou chaque aileron antéro-postérieur s'étendant à partir du bord interne dudit aileron médian externe. Cette configuration permet un appui particulièrement satisfaisant des tubérosités humérales.
- La partie métaphysaire de la prothèse comprend deux ailerons antéro-postérieurs, globalement symétriques l'un de l'autre par rapport au plan frontal. Ces ailerons permettent d'utiliser la prothèse de l'invention pour une épaule droite ou gauche et confèrent à cette partie métaphysaire une bonne stabilité dimensionnelle, notamment en torsion.
- La partie métaphysaire comprend deux branches, respectivement externe et interne, délimitant entre elles un évidement, la branche externe étant pourvue du ou de chaque aileron antéro-postérieur. Cet évidement permet la fusion osseuse à travers la partie métaphysaire de la prothèse.
- La branche externe est formée dudit aileron médian externe, et desdits deux ailerons antéro-postérieurs. Ce mode de réalisation est avantageux puisqu'il est de construction simple et qu'il permet à la prothèse de présenter une surface externe large de contact avec les tubérosités humérales. Dans ce cas, la branche externe présente avantageusement, en section transversale, une forme en T.
- Le bord libre du ou de chaque aileron est incliné par rapport au plan frontal, la largeur du ou de chaque aileron allant en croissant en direction de la collerette.

L'invention va être décrite ci-dessous, en référence aux dessins annexés, donnés uniquement à titre d'exemples non limitatifs et dans lesquels :
- la figure 1 est une vue schématique de dessus, représentant l'extrémité supérieure d'un humérus ;
- les figures 2 et 3 sont des vues respectivement de face et de côté d'une prothèse humérale conforme à l'invention ;
- la figure 4 est une coupe selon la ligne IV-IV à la figure 3 ;
- la figure 5 est une vue en perspective illustrant la prothèse des figures 2 et 3 implantée dans le canal médullaire d'un humérus, ainsi que des tubérosités humérales disposées au voisinage de cette prothèse, et
- la figure 6 est une vue de dessus, analogue à la figure 1, illustrant la prothèse une fois implantée, la calotte d'appui sur la glène étant omise.

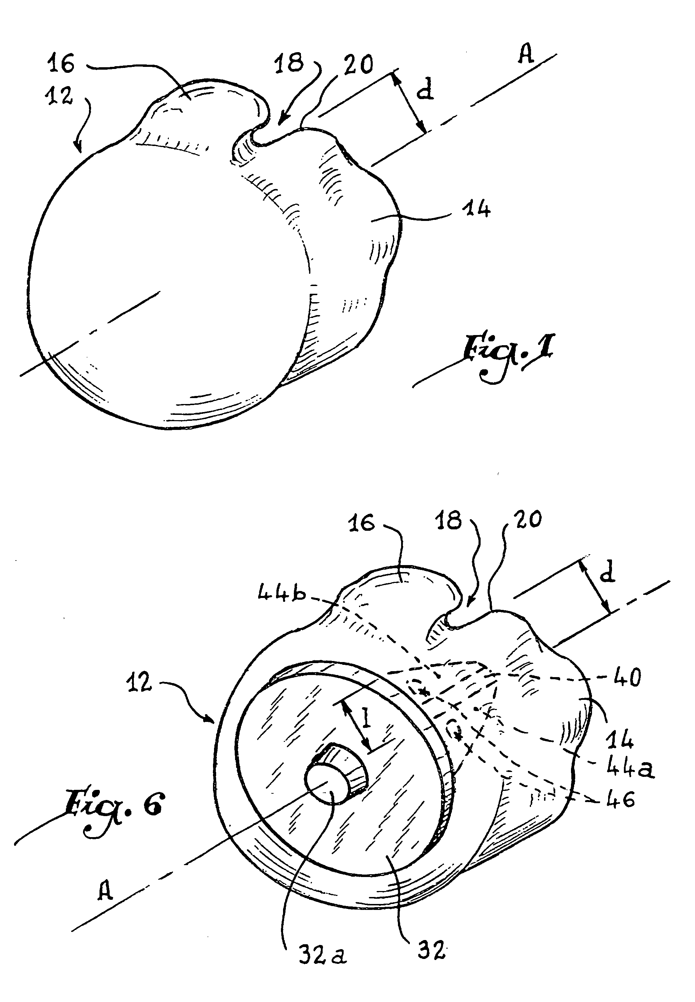

La figure 1 représente une extrémité supérieure d'un humérus 10, qui comprend une partie métaphysaire surmontée d'une tête humérale 12, coopérant avec la glène de l'épaule non représentée. Sur la partie métaphysaire sont formées une grande tubérosité 14 et une petite tubérosité 16 qui délimitent, au niveau de leur zone de jonction, une gorge 18, dénommée gouttière bicipitale.

Lors de la fracture de l'humérus 10, le bord postérieur 20 de la gouttière 18, adjacent à la grande tubérosité 14, constitue souvent le point d'amorce d'une telle fracture. On désigne par A l'axe central de la tête de l'humérus 12, et par d la distance séparant cet axe A et le bord postérieur 20 de la gouttière 18. L'axe A est déterminé, de façon connue, selon une méthode décrite dans l'article "Anatomic détermination of humeral head rétroversion : the relationship of the central axis of the humeral head to the bicipital groove", extrait de la publication "Journal of Shoulder and Elbow Surgery", dans le numéro de septembre-octobre 1993.

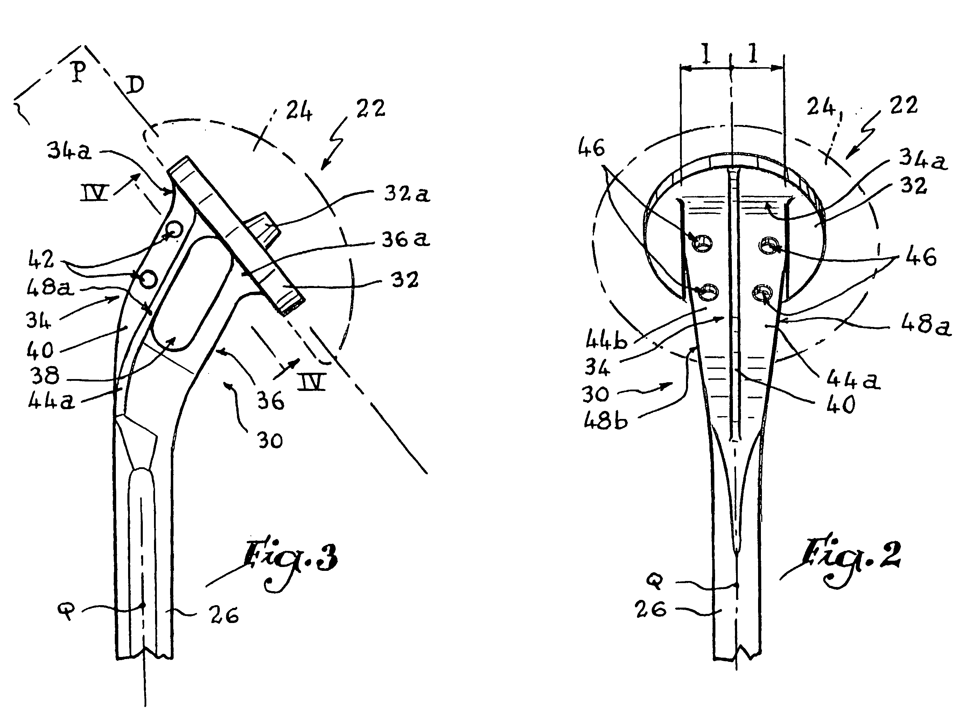

Les figures 2 à 4 représentent une prothèse conforme à l'invention, désignée dans son ensemble par la référence 22. Dans ce qui suit, les termes inférieur, supérieur, interne, externe, antérieur et postérieur doivent être compris comme se référant à une prothèse portée par un patient debout.

La prothèse 22 est destinée à supporter une calotte sensiblement hémisphérique 24, apte à coopérer avec la glène de l'épaule d'un patient.

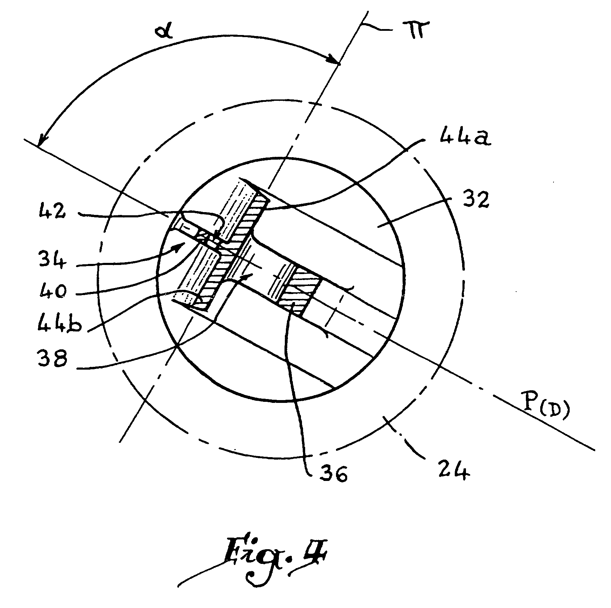

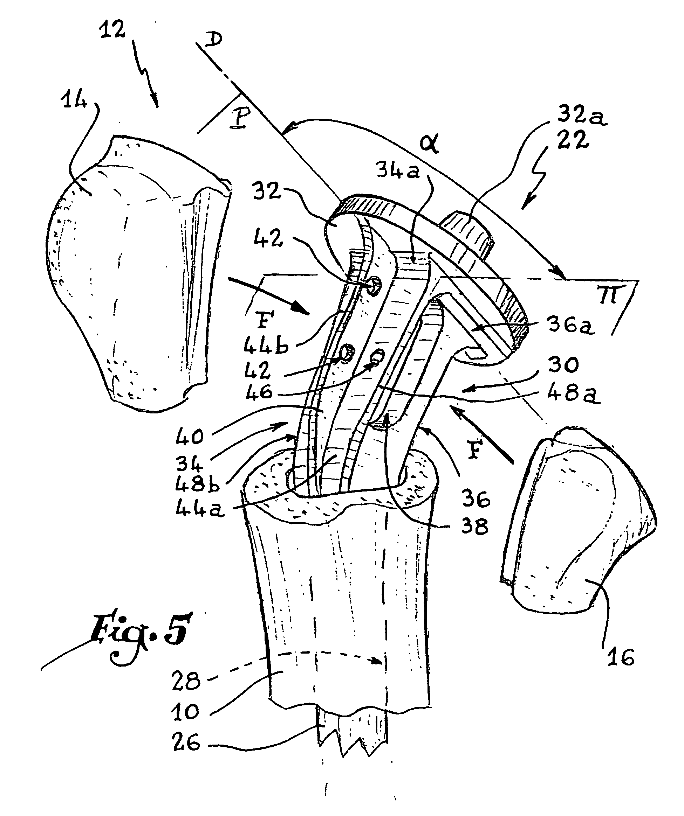

Cette prothèse comprend une queue 26 de section sensiblement circulaire, destinée à s'engager dans le canal huméral 28, comme le montre, en particulier, la figure 4. Cette queue 26 est prolongée par une partie métaphysaire 30, à laquelle est raccordée une collerette 32 propre à être solidarisée de manière amovible à la calotte 24 grâce à un pion 32a.

Le plan frontal P de la prothèse est le plan contenant l'axe central Q de la queue 26 et un axe médian ou diamètre D de la collerette 32. Le plan P est perpendiculaire au plan de la figure 2 et sa trace est confondue avec celle des axes D et Q sur cette figure. Le plan P est parallèle au plan de la figure 3. En pratique, le plan P est un plan de symétrie de la prothèse 22.

La partie métaphysaire 30 comprend des portions externe et interne, à savoir une branche externe 34 et une branche interne 36, qui se raccordent chacune à la face inférieure de la collerette 32, par des zones de raccord respectives 34a et 36a. Ces dernières sont situées sensiblement selon l'axe D. Une fois la prothèse implantée, l'axe D de la collerette est superposé à l'axe central A de la tête humérale 12.

Entre les branches 34 et 36 est défini un évidement 38 constituant un volume libre de rapprochement et de fusion des fragments d'os de la métaphyse. L'existence de ce volume libre permet la formation d'un pont osseux assurant l'ancrage efficace de cette prothèse dans l'humérus.

La branche interne 36 présente une section transversale rectangulaire, sensiblement constante sur toute la longueur de cette branche.

La branche externe 34 comprend un aileron médian 40, faisant saillie vers l'extérieur et présentant une épaisseur nettement inférieure à celle de la branche intérieure 36. Cet aileron médian externe 40 s'étend sensiblement selon le plan frontal P. Il est percé d'orifices 42 destiné au passage de fils de suture.

Deux ailerons antéro-postérieurs 44, à savoir un aileron latéral arrière 44a et un aileron latéral avant 44b, s'étendent, à partir du bord interne 40a de l'aileron médian 40, chacun selon un plan π dont on note α l'angle d'orientation par rapport au plan frontal P. L'angle α est d'environ 90°, c'est-à-dire que les ailerons antéro-postérieurs 44 sont globalement perpendiculaires à l'aileron médian 40. La branche externe 34 présente donc en section transversale visible à la figure 4, une forme en T.

Les ailerons antéro-postérieurs 44 permettent un repositionnement anatomique des tubérosités 14 et 16 et de la gouttière bicipitale 18 grâce à un appui sur l'un au moins des ailerons, comme représenté par les flèches F à la figure 5.

Bien que la valeur de 90° soit particulièrement avantageuse, des résultats satisfaisants ont pu être obtenus avec des ailerons antéro-postérieurs s'étendant dans un plan π dont l'angle par rapport au plan frontal est compris entre 45 et 135°.

Chacun des ailerons antéro-postérieurs 44a et 44b est percé d'orifices 46 permettant le passage de fils de suture.

Comme le montre en particulier la figure 2, la largeur des ailerons antéro-postérieurs 44 est croissante en direction de la collerette 32. Les bords libres respectifs 48a et 48b des ailerons 44a et 44b sont divergents de l'extérieur vers l'intérieur de l'épaule, c'est-à-dire inclinés par rapport au plan frontal P. La largeur maximale l des ailerons 44 est égale à la distance d séparant l'axe central A de la tête numérale 12 et le bord postérieur 20 de la gouttière bicipitale 18.

En effet, pour effectuer la synthèse des tubérosités humérales, le chirurgien dispose d'un repère dans la direction verticale, car le haut du trochiter doit se situer légèrement en dessous de la tête prophétique. Or, le trochiter peut tourner autour de l'axe de la calotte. L'invention permet de fournir au chirurgien un repère, afin de lui permettre de positionner correctement le trochiter. Ce repère est la position théorique de la gouttière bicipitale 18, déterminée par la largeur maximale l des ailerons antéro-postérieurs. Cette largeur correspond en effet à la distance anatomique moyenne mesurée entre l'axe central A et le bord postérieur 20 de la gouttière bicipitale 18.

Une fois la prothèse 22 mise en place et les tubérosités humérales 14 et 16 solidarisées au voisinage de cette prothèse, comme représenté à la figure 6, la fusion osseuse peut avoir lieu, cette fusion aboutissant à une reconstruction anatomique de l'os.

La largeur des ailerons, et notamment leur largeur maximale l peut être adaptée en fonction de la taille de la prothèse, c'est-à-dire, en particulier, de la position de la gouttière bicipitale. En pratique, cette largeur l est comprise entre 5,5 et 13 mm et l'on peut prévoir trois tailles de prothèses dont les ailerons antéro-postérieurs ont respectivement une largeur maximale l égale à 6, 9 ou 12 mm.

Dans le cas où les ailerons 44a et 44b ne sont pas perpendiculaires au plan frontal, leur largeur maximale l est choisie pour que sa projection sur un plan perpendiculaire à ce plan frontal corresponde à la distance d. Dans le cas représenté sur les figures, la largeur l est égale à sa projection sur le plan π.

## Revendications

1. Prothèse humérale (22), comprenant une queue (26) destinée à être ancrée dans le canal médullaire d'un humérus, une partie métaphysaire (30), prolongeant ladite queue vers le haut et dont la portion interne (36) est inclinée vers l'intérieur, ladite partie métaphysaire se raccordant à une collerette (32) de support d'une calotte (24) apte à coopérer avec la glène de l'épaule, **caractérisée en ce que** la portion externe (34) de la partie métaphysaire (30) est également inclinée vers l'intérieur, et cette portion externe (34) comprend au moins un aileron antéro-postérieur (44) d'appui des tubérosités humérales (14, 16), le ou chaque aileron s'étendant dans un plan (π) orienté selon un angle (a) compris entre 45° et 135° par rapport au plan frontal (P) de ladite prothèses, ledit plan frontal (P) contenant l'axe central (Q) de la queue (26).

2. Prothèse selon la revendication 1, **caractérisée en ce que** l'angle (α) d'orientation du plan (π) dudit aileron antéro-postérieur (44) par rapport audit plan frontal (P)est voisin de 90°.

3. Prothèse selon l'une des revendications 1 ou 2, **caractérisée en ce que** le ou chaque aileron antéro-postérieur (44) s'étend, à partir dudit plan frontal (P), avec une largeur maximale (l) telle que sa projection sur un plan perpendiculaire audit plan frontal (P) correspond sensiblement à la distance (d) séparant le bord postérieur (20) de la gouttière bicipitale (18) de la métaphyse humérale, par rapport à un axe central (A) de ladite métaphyse humérale (12).

4. Prothèse selon l'une quelconque des revendications précédentes, **caractérisée en ce que** ladite partie métaphysaire (30) comprend en outre, dans sa portion externe (34), un aileron médian externe (40) s'étendant globalement selon ledit plan frontal (P), le ou chaque aileron antéro-postérieur (44) s'étendant à partir du bord interne (40a) dudit aileron médian externe (40).

5. Prothèse selon l'une des revendications précédentes, **caractérisée en ce que** ladite partie métaphysaire (30) comprend deux ailerons antéro-postérieurs (44a, 44b), globalement symétriques l'un de l'autre par rapport audit plan frontal (P).

6. Prothèse selon l'une des revendications précédentes, **caractérisée en ce que** la partie métaphysaire (30) comprend deux branches, respectivement externe (34) et interne (36), délimitant entre elles un évidement (38), la branche externe (34) étant pourvue du ou de chaque aileron antéro-postérieur (44).

7. Prothèse selon les revendications 4 à 6, **caractérisée en ce que** ladite branche externe (34) est formée dudit aileron médian (40), et desdits deux ailerons antéro-postérieurs (44).

8. Prothèse selon la revendication 7, **caractérisée en ce que** ladite branche externe (34) présente, en section transversale, une forme en T.

9. Prothèse selon l'une des revendications précédentes, **caractérisée en ce que** le bord libre (48a, 48b) dudit ou de chaque aileron antéro-postérieur (44) est incliné par rapport audit plan frontal (P), la largeur dudit ou de chaque aileron allant croissant en direction de ladite collerette (32).

## Patentansprüche

1. Oberarmknochenprothese (22) mit einem Stiel (26), der in dem medullären Kanal eines Oberarmknochens verankert werden soll, einem Metaphysenteil (30), der den Stiel nach oben verlängert und dessen innerer Teil (36) nach Innen geneigt ist, wobei der Metaphysenteil an einen Tragflansch (32) für einen Kopf (24) anschließt, der mit der Gelenkpfanne der Schulter zusammenwirken kann, **dadurch gekennzeichnet, dass** der äußere Teil (34) des Metaphysenteils (30) ebenso nach innen geneigt ist und dieser äußere Teil (34) zumindest einen anterior-posterior-Steg (44) zur Anlage der Oberarmknochenhöcker (14, 16) umfasst, wobei der oder jeder Steg sich in einer Ebene (π) erstreckt, die in einem Winkel (α) zwischen 45° und 135° relativ zu der Frontalebene (P) der Prothese orientiert ist und die Frontalebene (P) die Mittelachse (Q) des Stiels (26) enthält.

2. Prothese nach Anspruch 1, **dadurch gekennzeichnet, dass** der Orientierungswinkel (α) der Ebene (π) des anterior-posterior-Stegs (44) relativ zur Frontalebene (P) nahe 90° liegt.

3. Prothese nach einem der Ansprüche 1 oder 2, **dadurch gekennzeichnet, dass** der oder jeder anterior-posterior-Steg (44) sich von der Frontalebene (P) aus mit einer solchen maximalen Breite (l) erstreckt, dass seine Projektion auf eine Ebene senkrecht zur Frontalebene (P) im Wesentlichen dem Abstand (d) entspricht, der den posterioren Rand (20) der Bizepsrinne (18) der Oberarmmetaphyse von einer Mittelachse (A) der Oberarmmetaphyse (12) trennt.

4. Prothese nach einem der vorhergehenden Ansprüche, **dadurch gekennzeichnet, dass** der Metaphysenteil (30) außerdem auf seinem äußeren Teil (34) einen äußeren Mittelsteg (40) umfasst, der sich im Wesentlichen entlang der Frontalebene (P) erstreckt, wobei der oder jeder anterior-posterior-Steg (44) sich von dem Innenrand (40a) des äußeren Mittelsteg (40) aus erstreckt.

5. Prothese nach einem der vorhergehenden Ansprüche, **dadurch gekennzeichnet, dass** der Metaphysenteil (30) zwei anterior-posterior-Stege (44a, 44b) umfasst, die relativ zu der Frontalebene (P) im Wesentlichen symmetrisch zueinander sind.

6. Prothese nach einem der vorhergehenden Ansprüche, **dadurch gekennzeichnet, dass** der Metaphysenteil (30) zwei Äste umfasst, einen äußeren (34) beziehungsweise einen inneren (36), die zwischen sich eine Ausnehmung (38) begrenzen, wobei der äußere Ast (34) mit dem oder jedem anterior-posterior-Steg (44) versehen ist.

7. Prothese nach den Ansprüche 4 bis 6, **dadurch gekennzeichnet, dass** der äußere Ast (34) von dem Mittelsteg (40) und den zwei anterior-posterior-Stegen (44) gebildet wird.

8. Prothese nach Anspruch 7, **dadurch gekennzeichnet, dass** der äußere Ast (34) einen T-förmigen Querschnitt aufweist.

9. Prothese nach einem der vorhergehenden Ansprüche, **dadurch gekennzeichnet, dass** der freie Rand (48a, 48b) des oder jedes anterior-posterior-Stegs (44) relativ zu der Frontalebene (P) geneigt ist, wobei die Breite des oder jedes Stegs in Richtung des Flanschs (32) zunimmt.

## Claims

1. Humeral prosthesis (22), comprising a shaft (26) designed to be anchored in the medullary cavity of a humerus, a metaphysary part (30) which prolongs said shaft upwardly and the inner portion (36) of which is inclined towards the interior, said metaphysary part being connected to a support collar (32) for a cap (24) adapted to cooperate with the shoulder socket, **characterised in that** the outer portion (34) of the metaphysary part (30) is also inclined towards the interior, and this outer portion (34) includes at least one anteroposterior rib (44) for supporting the humeral tuberosities (14, 16), the or each rib extending in a plane (π) oriented at an angle (α) of between 45° and 135° with respect to the frontal plane (P) of said prosthesis, said frontal plane (P) containing the centre axis (Q) of the shaft (26).

2. Prosthesis according to claim 1, **characterised in that** the angle (α) of orientation of the plane (π) of said anteroposterior rib (44) with respect to said frontal plane (P) is proximate to 90°.

3. Prosthesis according to either of claims 1 and 2, **characterised in that** the or each anteroposterior rib (44) extends, starting from said frontal plane (P), with a maximum width (l) such that its projection on a plane perpendicular to said frontal plane (P) corresponds substantially to the distance (d) between the posterior edge (20) of the lateral bicipital groove (18) and the humeral metaphysis, in relation to a centre axis (A) of said humeral metaphysis (12).

4. Prosthesis according to any one of the preceding claims, **characterised in that** said metaphysary part (30) additionally includes in its outer portion (34) an outer medial rib (40) extending substantially in said frontal plane (P), the or each anteroposterior rib (44) extending from the inner edge (40a) of said outer medial rib (40).

5. Prosthesis according to any one of the preceding claims, **characterised in that** said metaphysary part (30) includes two anteroposterior ribs (44a, 44b), substantially symmetrical with one another in relation to said frontal plane (P).

6. Prosthesis according to any one of the preceding claims, **characterised in that** the metaphysary part (30) includes two, respectively outer (34) and inner (36), branches delimiting between them a cut-out (38), the outer branch (34) being provided with the or each anteroposterior rib (44).

7. Prosthesis according to claims 4 to 6, **characterised in that** said outer branch (34) is formed by said medial rib (40) and by said two anteroposterior ribs (44).

8. Prosthesis according to claim 7, **characterised in that** said outer branch (34) is T-shaped in cross-section.

9. Prosthesis according to any one of the preceding claims, **characterised in that** the free edge (48a, 48b) of said or each anteroposterior rib (44) is inclined with respect to said frontal plane (P), the width of said or each rib increasing in the direction towards said collar (32).
